# EUROPEAN PATENT APPLICATION

(11) **EP 4 091 548 A1**
(43) Date of publication of application: **23.11.2022**
(21) Application number: 20921112.7
(22) Date of filing: 26.10.2020
(51) Int. Cl.: A61B 5/318

(54) **SIGNAL MEASUREMENT METHOD AND DEVICE**

(30) Priority: 29.02.2020 CN 202010132725
(71) Applicant: Huawei Technologies Co., Ltd., Shenzhen, Guangdong 518129 (CN)
(72) Inventor: LIU, Xiangyu, Shenzhen, Guangdong 518129 (CN); YANG, Bin, Shenzhen, Guangdong 518129 (CN); ZHANG, Jie, Shenzhen, Guangdong 518129 (CN); CHEN, Yixin, Shenzhen, Guangdong 518129 (CN)
(74) Representative: Pfenning, Meinig & Partner mbB
(86) International application number: PCT/CN2020/123695
(87) International publication number: WO 2021/169339

(57) **Abstract**

A signal measurement method and a signal measurement apparatus (1000) are provided. A mode selection switch (11) may be used to enable a multi-lead measurement mode, to obtain signals of a plurality of leads and a status of a user. When quality of the signals of the plurality of leads is good or the user is in a static state, extracted features of the signals of the plurality of leads are output. When the signals of the plurality of leads are poor and the user is in a moving state, the mode selection switch (11) is used to switch to a single-lead mode with right leg drive, to obtain a signal of a single lead. In addition, a common-mode signal is eliminated from the signal of the single lead by using a negative feedback of a right leg drive electrode, and a feature of the signal of the single lead on which elimination processing is performed is output. Automatic switching between the multi-lead measurement mode and the single-lead measurement mode with the right leg drive is implemented by determining the quality of the signals and a motion status of the user, and a signal with high quality can be output, to accurately measure an electrocardio signal of the user.

## Description

This application claims priority to Chinese Patent Application No. 202010132725.1, filed with the China National Intellectual Property Administration on February 29, 2020 and entitled "SIGNAL MEASUREMENT METHOD AND APPARATUS", which is incorporated herein by reference in its entirety.

### TECHNICAL FIELD

This application relates to the field of medical electronic technologies, and in particular, to a signal measurement method and apparatus.

### BACKGROUND

Prevalence of cardiovascular diseases in China is continuously increasing, and a mortality rate of the cardiovascular diseases ranks first, and is higher than that of tumors and other diseases.

Electrocardiography (ECG) is most common testing means in cardiology and is a best method for measuring and diagnosing the cardiovascular diseases. However, a medical-standard 12-lead electrocardiograph is not suitable for a family or an individual due to a large size and a testing site limitation.

Currently, a wearable device, for example, an electrocardiogram watch or wristband is launched. Three electrodes are disposed in a watch body to measure electric potential between a left hand and a right hand, so that an ECG of a lead I can be measured. A specific measurement manner is that one or two electrodes disposed on the back of the watch (wristband) are in contact with skin of a wrist by wearing the watch (wristband), and a finger of the other hand actively touches a remaining electrode, to form measurement of the lead I. However, the measurement requires collaboration of the left hand and the right hand, and it is difficult to implement real-time measurement. In addition, only single-lead measurement can be formed between the left hand and the right hand.

In another solution, two or three electrodes are disposed on a headset, and real-time ECG measurement can be implemented when the headset is worn. However, a current solution in which an electrode is disposed on a headset to perform ECG measurement is currently a single-lead solution, and little information is obtained.

In addition, currently, there is no solution about how to ensure signal quality of an electrocardiogram when a user is in a moving state during static measurement of electrocardiogram information.

In view of this, how to improve quality of a measured electrocardio signal is a problem that needs to be resolved in this application.

### SUMMARY

This application provides a signal measurement method and apparatus, to improve quality of a measured signal.

According to a first aspect, a signal measurement method is provided, applied to a signal measurement apparatus. The method includes: enabling a multi-lead measurement mode, and obtaining signals of a plurality of leads and a status of a user; extracting features of the signals of the plurality of leads, and obtaining quality of the signals of the plurality of leads based on the features of the signals of the plurality of leads; and outputting the extracted features of the signals of the plurality of leads if the quality of the signals of the plurality of leads is greater than a first threshold or the user is in a static state; or switching to a single-lead mode with right leg drive if the quality of the signals of the plurality of leads is less than or equal to a first threshold and the user is in a moving state, and obtaining a signal of a single lead; eliminating a common-mode signal from the signal of the single lead; and extracting and outputting a feature of the signal of the single lead on which elimination processing is performed.

In this aspect, automatic switching between a multi-lead ECG measurement mode and a single-lead ECG measurement mode with a right leg drive electrode of a three-electrode ECG system is implemented by determining the quality of the signals and a motion status of the user. When the quality of the signals of the plurality of leads is good, the extracted features of the signals of the plurality of leads are output. Alternatively, the feature of the signal of the single lead on which the elimination processing is performed is extracted and output only after the common-mode signal is eliminated from the signal of the single lead, so that an electrocardio signal with higher quality can be obtained, to accurately measure an electrocardio signal of the user.

In a possible implementation, the signal measurement apparatus includes a left ear electrode, a right ear electrode, a neck electrode, preamplifiers respectively corresponding to the three electrodes, a Wilson network circuit, a common-mode generation circuit, a mode selection switch, and a processor. The left ear electrode is connected to a left ear measurement assembly, the right ear electrode is connected to a right ear measurement assembly, and the mode selection switch is connected to a neck measurement assembly. The common-mode generation circuit includes a right leg drive electrode and a common-mode amplifier. The left ear electrode, the right ear electrode, and the neck electrode are respectively and separately connected to positive electrodes of the corresponding preamplifiers and the Wilson network circuit, a central electrical terminal of the Wilson network circuit is separately connected to negative electrodes of the preamplifiers of the three electrodes, and the preamplifiers respectively corresponding to the three electrodes are connected to the processor. The central electrical terminal of the Wilson network circuit is further connected to a negative electrode of the common-mode amplifier. The enabling a multi-lead measurement mode includes: controlling the mode selection switch to be connected to the neck electrode, to enable the multi-lead measurement mode.

In this implementation, the signal measurement apparatus is simple in circuit implementation, and convenient in measurement mode switching.

In another possible implementation, the obtaining signals of a plurality of leads includes: measuring a signal between the left ear electrode and the right ear electrode, to obtain a signal of a lead I; measuring a signal between the right ear electrode and the neck electrode, to obtain a signal of a lead II; measuring a signal between the left ear electrode and the neck electrode, to obtain a signal of a lead III; and obtaining a signal of a lead aVR, a signal of a lead aVL, and a signal of a lead avF based on the signal of the lead I, the signal of the lead II, and the signal of the lead III.

In this implementation, signals of six leads can be obtained by using the three measurement electrodes and a Wilson network. A measurement operation of the user is simple.

In still another possible implementation, the obtaining a signal of a lead aVR, a signal of a lead aVL, and a signal of a lead avF based on the signal of the lead I, the signal of the lead II, and the signal of the lead III includes: The signal of the lead aVR satisfies: lead aVR=RE-0.5×(LE+N), the signal of the lead aVL satisfies: lead aVL=LE-0.5×(N+RE), and the signal of the lead avF satisfies: lead avF=N-0.5×(LE+RE). RE is a signal of the right ear electrode, LE is a signal of the left ear electrode, and N is a signal of the neck electrode.

In still another possible implementation, the extracting features of the signals of the plurality of leads includes: obtaining an average value of the signals of the plurality of leads; and obtaining, as a feature of a signal of each lead based on the signal of each lead in the signals of the plurality of leads and the average value of the signals of the plurality of leads, a correlation coefficient corresponding to the signal of each lead.

In this implementation, the correlation coefficient corresponding to the signal of the lead can accurately represent the feature of the signal of the lead.

In still another possible implementation, the method further includes: obtaining, as the quality of the signals of the plurality of leads based on the correlation coefficient corresponding to the signal of each lead, an average value of the correlation coefficients corresponding to the signals of the plurality of leads; and performing the step of extracting the features of the signals of the plurality of leads if the average value of the correlation coefficients corresponding to the signals of the plurality of leads is greater than the first threshold or an acceleration signal is less than or equal to a second threshold; or performing the step of switching to the single-lead mode with the right leg drive if the average value of the correlation coefficients corresponding to the signals of the plurality of leads is less than or equal to the first threshold and an acceleration signal is greater than or equal to a third threshold.

In this implementation, when the quality of the signals of the plurality of leads is poor and the user is in the moving state, the signals of the plurality of leads obtained through measurement are unstable and cannot be output as a measurement result. Therefore, switching to a single-lead measurement mode with a right leg drive can eliminate the common-mode signal from the signal of the single lead by using a negative feedback of the right leg drive electrode, to obtain a signal of a single lead with good quality.

In still another possible implementation, the method further includes: screening a signal that is greater than a fourth threshold from the signals of the plurality of leads; and extracting a feature of the screened signal.

In this implementation, a signal with good signal quality can be screened from the signals of the plurality of leads based on a requirement, and then the feature of the screened signal is extracted, to improve quality of an output result.

In still another possible implementation, the switching to a single-lead mode with right leg drive if the signals of the plurality of leads are less than or equal to a first threshold and the user is in a moving state, and obtaining a signal of a single lead includes: if the signals of the plurality of leads are less than or equal to the first threshold and the user is in the moving state, controlling the mode selection switch to switch to the right leg drive electrode, to enable the single-lead mode. The obtaining a signal of a single lead includes: obtaining the signal of the single lead based on the signal of the left ear electrode, the signal of the right ear electrode, and a signal of the central electric terminal. The eliminating a common-mode signal from the signal of the single lead includes: obtaining, based on the signal of the central electric terminal and a reference level signal, a common-mode signal of the user by using a negative feedback of the right leg drive electrode; and eliminating the common-mode signal from the signal of the single lead.

In this implementation, when the quality of the signals of the plurality of leads is poor, and the user is in the moving state, the signals of the plurality of leads obtained through measurement are unstable and cannot be output as the measurement result. Therefore, switching to a single-lead measurement mode with the right leg drive can eliminate the common-mode signal from the signal of the single lead by using the negative feedback of the right leg drive electrode, to obtain the signal of the single lead with good quality.

In still another possible implementation, the obtaining a status of a user includes: obtaining the acceleration signal; and if the acceleration signal is less than or equal to the second threshold, determining that the user is in the static state; or if the acceleration signal is greater than or equal to the third threshold, determining that the user is in the moving state.

In this implementation, the status of the user has great impact on signal measurement. Therefore, an accurate status of the user can be obtained by determining the acceleration signal.

According to a second aspect, a signal measurement apparatus is provided. The apparatus includes: a mode selection switch, configured to enable a multi-lead measurement mode; a signal obtaining module, configured to obtain signals of a plurality of leads and a status of a user; a feature extraction module, configured to extract features of the signals of the plurality of leads; a signal quality obtaining module, configured to obtain quality of the signals of the plurality of leads based on the features of the signals of the plurality of leads; an output module, configured to output the extracted features of the signals of the plurality of leads if the signals of the plurality of leads are greater than a first threshold or the user is in a static state, where the mode selection switch is further configured to switch to a single-lead mode with right leg drive if the signals of the plurality of leads are less than or equal to a first threshold and the user is in a moving state, and the signal obtaining module is further configured to obtain a signal of a single lead; and a common-mode signal elimination module, configured to eliminate a common-mode signal from the signal of the single lead. The feature extraction module is further configured to extract a feature of the signal of the single lead on which elimination processing is performed. The output module is further configured to output the extracted feature of the signal of the single lead.

In a possible implementation, the signal obtaining module includes a left ear electrode, a right ear electrode, a neck electrode, preamplifiers respectively corresponding to the three electrodes, a Wilson network circuit, and a processor. The common-mode signal elimination module includes a right leg drive electrode and a common-mode amplifier. The left ear electrode is connected to a left ear measurement assembly, the right ear electrode is connected to a right ear measurement assembly, and the mode selection switch is connected to a neck measurement assembly. The left ear electrode, the right ear electrode, and the neck electrode are respectively and separately connected to positive electrodes of the corresponding preamplifiers and the Wilson network circuit, a central electrical terminal of the Wilson network circuit is separately connected to negative electrodes of the preamplifiers of the three electrodes, and the preamplifiers respectively corresponding to the three electrodes are connected to the processor. The central electrical terminal of the Wilson network circuit is further connected to a negative electrode of the common-mode amplifier. The module selection switch is configured to connect to the neck electrode, to enable the multi-lead measurement mode.

In another possible implementation, the signal obtaining module is configured to: measure a signal between the left ear electrode and the right ear electrode, to obtain a signal of a lead I; measure a signal between the right ear electrode and the neck electrode, to obtain a signal of a lead II; measure a signal between the left ear electrode and the neck electrode, to obtain a signal of a lead III; and obtain a signal of a lead aVR, a signal of a lead aVL, and a signal of a lead avF based on the signal of the lead I, the signal of the lead II, and the signal of the lead III.

In still another possible implementation, the signal of the lead aVR satisfies: lead aVR=RE-0.5×(LE+N), the signal of the lead aVL satisfies: lead aVL=LE-0.5×(N+RE), and the signal of the lead avF satisfies: lead avF=N-0.5×(LE+RE). RE is a signal of the right ear electrode, LE is a signal of the left ear electrode, and N is a signal of the neck electrode.

In still another possible implementation, the feature extraction module is configured to obtain an average value of the signals of the plurality of leads; and obtain, as a feature of a signal of each lead based on the signal of each lead in the signals of the plurality of leads and the average value of the signals of the plurality of leads, a correlation coefficient corresponding to the signal of each lead.

In still another possible implementation, the signal quality obtaining module is configured to obtain, as the quality of the signals of the plurality of leads based on the correlation coefficient corresponding to the signal of each lead, an average value of the correlation coefficients corresponding to the signals of the plurality of leads. The output module is configured to output the features of the signals of the plurality of leads if the average value of the correlation coefficients corresponding to the signals of the plurality of leads is greater than the first threshold or an acceleration signal is less than a second threshold. The mode selection switch is configured to switch to the single-lead mode with the right leg drive if the average value of the correlation coefficients corresponding to the signals of the plurality of leads is less than or equal to the first threshold and the acceleration signal is greater than or equal to the second threshold.

In still another possible implementation, the apparatus further includes a signal screening module, configured to screen a signal that is greater than a fourth threshold from the signals of the plurality of leads. The feature extraction module is configured to extract a feature of the screened signal.

In still another possible implementation, the mode selection switch is configured to switch to the right leg drive electrode if the signals of the plurality of leads are less than or equal to the first threshold and the user is in the moving state, to enable the single-lead mode. The signal obtaining module is configured to obtain the signal of the single lead based on the signal of the left ear electrode, the signal of the right ear electrode, and a signal of the central electric terminal. The common-mode signal elimination module is configured to obtain, based on the signal of the central electric terminal and a reference level signal, a common-mode signal of the user by using a negative feedback of the right leg drive electrode, and eliminate the common-mode signal from the signal of the single lead.

In still another possible implementation, the signal obtaining module is further configured to obtain the acceleration signal. The apparatus further includes a determining module, configured to: if the acceleration signal is less than or equal to the second threshold, determine that the user is in the static state. The determining module is further configured to: if the acceleration signal is greater than or equal to a third threshold, determine that the user is in the moving state.

According to a third aspect, a signal measurement apparatus is provided, including an input apparatus, an output apparatus, a memory, and a processor. The memory stores program instructions, and the processor is configured to invoke the program instructions to perform the following operations:
enabling a multi-lead measurement mode, and obtaining signals of a plurality of leads and a status of a user; extracting features of the signals of the plurality of leads, and obtaining quality of the signals of the plurality of leads based on the features of the signals of the plurality of leads; and controlling the output apparatus to output the extracted features of the signals of the plurality of leads if the quality of the signals of the plurality of leads is greater than a first threshold or the user is in a static state; or switching to a single-lead mode with right leg drive if the quality of the signals of the plurality of leads is less than or equal to a first threshold and the user is in a moving state, and obtaining a signal of a single lead; eliminating a common-mode signal from the signal of the single lead; and extracting a feature of the signal of the single lead on which elimination processing is performed, and controlling the output apparatus to output the extracted feature of the signal of the single lead.

In a possible implementation, the processor is further configured to perform the following operation: controlling a mode selection switch to be connected to a neck electrode, to enable the multi-lead measurement mode.

In another possible implementation, that the processor performs the operation of obtaining signals of a plurality of leads includes: measuring a signal between a left ear electrode and a right ear electrode, to obtain a signal of a lead I; measuring a signal between the right ear electrode and the neck electrode, to obtain a signal of a lead II; measuring a signal between the left ear electrode and the neck electrode, to obtain a signal of a lead III; and obtaining a signal of a lead aVR, a signal of a lead aVL, and a signal of a lead avF based on the signal of the lead I, the signal of the lead II, and the signal of the lead III.

In still another possible implementation, that the processor performs the operation of obtaining a signal of a lead aVR, a signal of a lead aVL, and a signal of a lead avF based on the signal of the lead I, the signal of the lead II, and the signal of the lead III includes: The signal of the lead aVR satisfies: lead aVR=RE-0.5×(LE+N), the signal of the lead aVL satisfies: lead aVL=LE-0.5×(N+RE), and the signal of the lead avF satisfies: lead avF=N-0.5×(LE+RE). RE is a signal of the right ear electrode, LE is a signal of the left ear electrode, and N is a signal of the neck electrode.

In still another possible implementation, that the processor performs the operation of extracting features of the signals of the plurality of leads includes: obtaining an average value of the signals of the plurality of leads; and obtaining, as a feature of a signal of each lead based on the signal of each lead in the signals of the plurality of leads and the average value of the signals of the plurality of leads, a correlation coefficient corresponding to the signal of each lead.

In still another possible implementation, the processor further performs the following operations: obtaining, as the quality of the signals of the plurality of leads based on the correlation coefficient corresponding to the signal of each lead, an average value of the correlation coefficients corresponding to the signals of the plurality of leads; and performing the operation of extracting the features of the signals of the plurality of leads if the average value of the correlation coefficients corresponding to the signals of the plurality of leads is greater than the first threshold or an acceleration signal is less than or equal to a second threshold; or performing the operation of switching to the single-lead mode with the right leg drive if the average value of the correlation coefficients corresponding to the signals of the plurality of leads is less than or equal to the first threshold and an acceleration signal is greater than or equal to a third threshold.

In still another possible implementation, the processor further performs the following operations: screening a signal that is greater than a fourth threshold from the signals of the plurality of leads; and extracting a feature of the screened signal.

In still another possible implementation, that the processor performs the operation of switching to a single-lead mode with the right leg drive if the signals of the plurality of leads are less than or equal to a first threshold and the user is in a moving state, and obtaining a signal of a single lead includes: if the signals of the plurality of leads are less than or equal to the first threshold and the user is in the moving state, controlling the mode selection switch to switch to a right leg drive electrode, to enable the single-lead mode. That the processor performs the operation of obtaining a signal of a single lead includes: obtaining the signal of the single lead based on the signal of the left ear electrode, the signal of the right ear electrode, and a signal of a central electric terminal. That the processor performs the operation of eliminating a common-mode signal from the signal of the single lead includes: obtaining, based on the signal of the central electric terminal and a reference level signal, a common-mode signal of the user by using a negative feedback of the right leg drive electrode; and eliminating the common-mode signal from the signal of the single lead.

In still another possible implementation, that the processor performs the operation of obtaining a status of a user includes: obtaining the acceleration signal; and if the acceleration signal is less than or equal to the second threshold, determining that the user is in the static state; or if the acceleration signal is greater than or equal to the third threshold, determining that the user is in the moving state.

According to a fourth aspect, a computer-readable storage medium is provided. The computer-readable storage medium stores instructions. When the instructions are run on a computer, the computer is enabled to perform the method according to any one of the first aspect or the possible implementations of the first aspect.

According to a fifth aspect, a computer program product including instructions is provided. When the computer program product runs on a computer, the computer is enabled to perform the method according to any one of the first aspect or the possible implementations of the first aspect.

### BRIEF DESCRIPTION OF DRAWINGS

FIG. 1 is a schematic diagram of a structure of a neckband headset according to an embodiment of this application;
FIG. 2 is a schematic diagram of a structure of a signal measurement apparatus according to an embodiment of this application;
FIG. 3 is a schematic flowchart of a signal measurement method according to an embodiment of this application;
FIG. 4A and FIG. 4B are a schematic flowchart of another signal measurement method according to an embodiment of this application;
FIG. 5 is a schematic diagram of measurement in a multi-lead measurement mode by using the neckband headset shown in FIG. 1;
FIG. 6 is a schematic diagram of an example of a structure of an internal circuit of a signal measurement apparatus in a multi-lead measurement mode;
FIG. 7 is a schematic diagram of an example of a structure of an internal circuit of a signal measurement apparatus in a single-lead measurement mode;
FIG. 8 is a schematic diagram of a scenario in which a user performs measurement by using a signal measurement apparatus; and
FIG. 9 is a schematic diagram of a structure of another signal measurement apparatus according to an embodiment of this application.

### DESCRIPTION OF EMBODIMENTS

The following describes embodiments of this application with reference to the accompanying drawings in embodiments of this application.

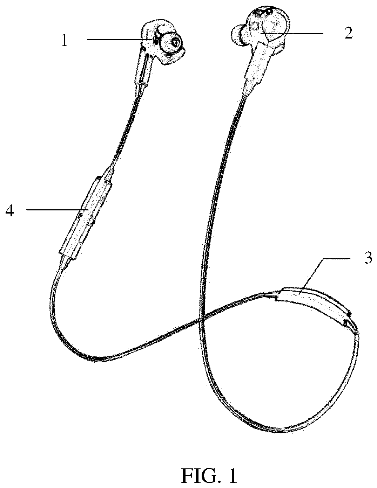

Currently, there is such a solution: Two or three electrodes are disposed on a headset, to implement real-time ECG measurement when the headset is worn. However, a current solution in which an electrode is disposed on a headset to perform ECG measurement is currently a single-lead solution, and little information is obtained. FIG. 1 is a schematic diagram of a structure of a neckband headset according to an embodiment of this application. On the neckband headset, an electrode disposing manner is as follows: A measurement assembly 1 (connected to an LE electrode) on a housing part of a left ear (left ear, LE) earpiece and a measurement assembly 2 (connected to a RE electrode) on a housing part of a right ear (right ear, RE) earpiece are respectively configured to be in contact with skin of auricle of the left and right ears of a user. However, without limitation, the housings of the left earpiece and the right earpiece may be implemented in a structure, for example, an ear mount. A neck (neck, N) measurement assembly 3 (connected to an N electrode) is used as a third electrode in a six-lead solution and a "right leg drive electrode" in a single-lead solution. A processing circuit 4 includes an analog front end (analog front end, AFE), an accelerometer (accelerometer, ACC), and a micro control unit (micro control unit, MCU). The AFE includes the foregoing electrodes, an operational amplifier (amplifier, AMP), an analog-to-digital converter (analog-to-digital converter, ADC), and the like. In an embodiment, the neckband headset may process a measured signal by the neckband headset. In another embodiment, after measuring a signal of each electrode, the neckband headset may alternatively send the signal to a terminal wirelessly connected to the headset, and the terminal performs processing. The circuit 4 may further include a communication module, for example, a Bluetooth transmission module. A communication manner of the communication module may be a wireless or wired connection manner. The terminal may be a mobile phone, a smartwatch, a smart band, a computer, or the like.

The neckband headset in this embodiment may work in a multi-lead measurement mode and a single-lead measurement mode. Specifically, signals of a plurality of leads may be obtained after signals obtained through measurement by electrodes 1, 2, and 3 are processed. When quality of the signals of the plurality of leads is good or the user is in a relatively static state, features of the signals of the plurality of leads are extracted. If the quality of the signals of the plurality of leads is poor or the user is in a moving state, the neckband headset may be switched to the single-lead measurement mode. In this case, a neck electrode is used as the right leg drive electrode, and common-mode components of electrical signals on surfaces of a left ear electrode and a right ear electrode are transmitted to the right leg drive electrode. A common-mode voltage of a human body is forced to approach a reference level by using a negative feedback of right leg drive, so that centers of inputs of all the electrodes are located at a center of an input range, to reduce common-mode noise of a system and improve signal quality of a signal of a single lead.

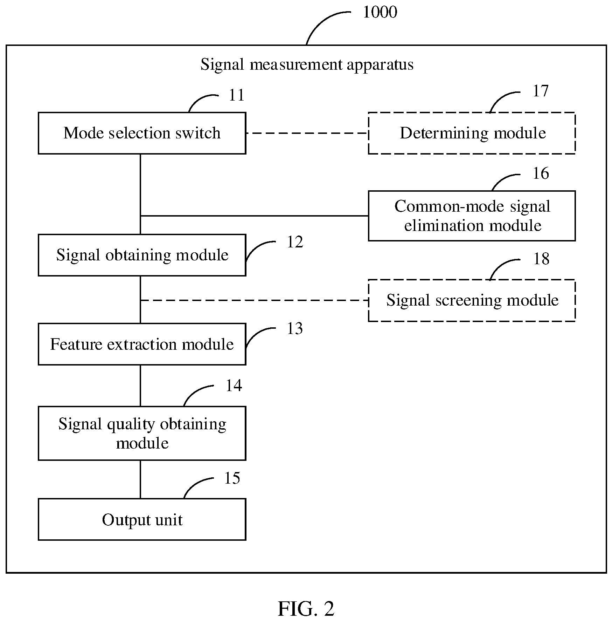

FIG. 2 is a schematic diagram of a structure of a signal measurement apparatus according to an embodiment of this application. The signal measurement apparatus 1000 includes: a mode selection switch 11, configured to enable a multi-lead measurement mode; a signal obtaining module 12, configured to obtain signals of a plurality of leads and a status of a user; a feature extraction module 13, configured to extract features of the signals of the plurality of leads; a signal quality obtaining module 14, configured to obtain quality of the signals of the plurality of leads based on the features of the signals of the plurality of leads; an output module 15, configured to output the extracted features of the signals of the plurality of leads if the quality of the signals of the plurality of leads is greater than a first threshold or the user is in a static state, where the mode selection switch 11 is further configured to switch to a single-lead mode with right leg drive if the quality of the signals of the plurality of leads is less than or equal to the first threshold and the user is in a moving state, and the signal obtaining module 12 is further configured to obtain a signal of a single lead; and a common-mode signal elimination module 16, configured to eliminate a common-mode signal from the signal of the single lead. The feature extraction module 13 is further configured to extract a feature of the signal of the single lead on which elimination processing is performed. The output module 15 is further configured to output the extracted feature of the signal of the single lead.

In a possible implementation, the signal obtaining module 12 includes a left ear electrode, a right ear electrode, a neck electrode, preamplifiers respectively corresponding to the three electrodes, a Wilson network circuit, and a processor. The common-mode signal elimination module 16 includes a right leg drive electrode and a common-mode amplifier. The left ear electrode is connected to a left ear measurement assembly, the right ear electrode is connected to a right ear measurement assembly, and the mode selection switch 11 is connected to a neck measurement assembly. The left ear electrode, the right ear electrode, and the neck electrode are respectively and separately connected to positive electrodes of the corresponding preamplifiers and the Wilson network circuit, a central electrical terminal of the Wilson network circuit is separately connected to negative electrodes of the preamplifiers of the three electrodes, and the preamplifiers respectively corresponding to the three electrodes are connected to the processor. The central electrical terminal of the Wilson network circuit is further connected to a negative electrode of the common-mode amplifier. The module selection switch 11 is configured to connect to the neck electrode, to enable the multi-lead measurement mode.

In another possible implementation, the signal obtaining module 12 is configured to: measure a signal between the left ear electrode and the right ear electrode, to obtain a signal of a lead I; measure a signal between the right ear electrode and the neck electrode, to obtain a signal of a lead II; measure a signal between the left ear electrode and the neck electrode, to obtain a signal of a lead III; and obtain a signal of a lead aVR, a signal of a lead aVL, and a signal of a lead avF based on the signal of the lead I, the signal of the lead II, and the signal of the lead III.

In still another possible implementation, the signal of the lead aVR satisfies: lead aVR=RE-0.5×(LE+N), the signal of the lead aVL satisfies: lead aVL=LE-0.5×(N+RE), and the signal of the lead avF satisfies: lead avF=N-0.5×(LE+RE). RE is a signal of the right ear electrode, LE is a signal of the left ear electrode, and N is a signal of the neck electrode.

In still another possible implementation, the feature extraction module 13 is configured to obtain an average value of the signals of the plurality of leads; and obtain, as a feature of a signal of each lead based on the signal of each lead in the signals of the plurality of leads and the average value of the signals of the plurality of leads, a correlation coefficient corresponding to the signal of each lead.

In still another possible implementation, the signal quality obtaining module 14 is configured to obtain, based on the correlation coefficient corresponding to the signal of each lead, an average value of the correlation coefficients corresponding to the signals of the plurality of leads. The output module 15 is configured to output the extracted features of the signals of the plurality of leads if the average value of the correlation coefficients corresponding to the signals of the plurality of leads is greater than the first threshold or an acceleration signal is less than a second threshold. The mode selection switch 11 is configured to switch to the single-lead mode with the right leg drive if the average value of the correlation coefficients corresponding to the signals of the plurality of leads is less than or equal to the first threshold and the acceleration signal is greater than or equal to the second threshold.

In still another possible implementation, the apparatus further includes a signal screening module 18, configured to screen a signal that is greater than a fourth threshold from the signals of the plurality of leads. The feature extraction module 13 is configured to extract a feature of the screened signal.

In still another possible implementation, the mode selection switch 11 is configured to switch to the right leg drive electrode if the signals of the plurality of leads are less than or equal to the first threshold and the user is in the moving state, to enable the single-lead mode. The signal obtaining module 12 is configured to obtain the signal of the single lead based on the signal of the left ear electrode, the signal of the right ear electrode, and a signal of the central electric terminal. The common-mode signal elimination module 16 is configured to obtain, based on the signal of the central electric terminal and a reference level signal, a common-mode signal of the user by using a negative feedback of the right leg drive electrode, and eliminate the common-mode signal from the signal of the single lead.

In still another possible implementation, the signal obtaining module 12 is further configured to obtain the acceleration signal. The apparatus further includes a determining module 17, configured to: if the acceleration signal is less than or equal to the second threshold, determine that the user is in the static state. The determining module is further configured to: if the acceleration signal is greater than or equal to a third threshold, determine that the user is in the moving state.

The following describes a signal measurement process in detail with reference to the signal measurement apparatus shown in FIG. 2.

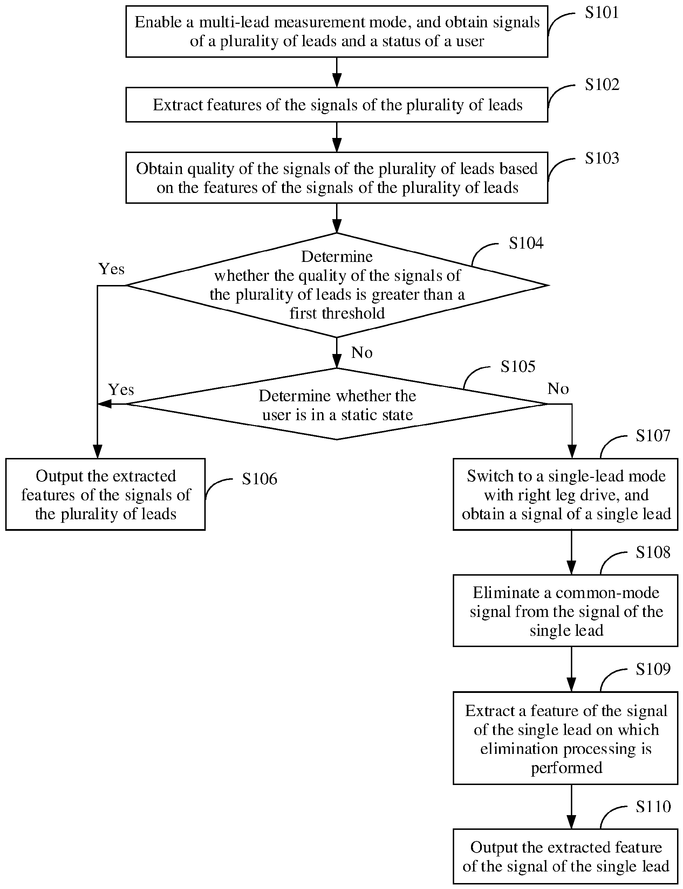

FIG. 3 is a schematic flowchart of a signal measurement method according to an embodiment of this application. The method may include the following steps.

S101: A mode selection switch enables a multi-lead measurement mode, and a signal obtaining module obtains signals of a plurality of leads and a status of a user.

In this embodiment, a signal measurement apparatus includes a plurality of measurement electrodes (for example, the electrodes LE, RE, and N of the neckband headset shown in FIG. 1), and can measure signals of a plurality of measurement assemblies (LE, RE, and N measurement assemblies). The signal measurement apparatus further includes a right leg drive electrode. A right leg drive circuit including the right leg drive electrode implements essentially a negative feedback. A function of the right leg drive circuit is usually used to remove a common-mode signal input to an amplifier, to improve a common-mode rejection ratio (common-mode rejection ratio, CMRR). The common-mode signal is reversely amplified and connected to a human body, to eliminate a common mode. Right leg drive is mainly used to reduce a common-mode voltage in bioelectric collection. The right leg drive is a necessary method to reduce common-mode interference.

One end of the mode selection unit is connected to one of the measurement assemblies (for example, the N measurement assembly), and another end of the mode selection unit may be connected to one measurement electrode (for example, the N electrode), or may be connected to the right leg drive electrode. When the another end of the mode selection unit is connected to the measurement electrode (for example, the N electrode), the signal measurement apparatus enables the multi-lead measurement mode. When the another end of the mode selection unit is connected to the right leg drive electrode, the signal measurement apparatus enables a single-lead measurement mode.

The mode selection unit may enable the multi-lead measurement mode by receiving instructions sent by a terminal or based on a default configuration of the signal measurement apparatus. Specifically, the instructions sent by the terminal are used to instruct the signal measurement apparatus to enable the multi-lead measurement mode. Alternatively, the signal measurement apparatus configures, by default, that the multi-lead measurement mode is enabled when the signal measurement apparatus is powered on. Further, if the signal measurement apparatus is in a single-lead mode before the multi-lead measurement mode is enabled, the signal measurement apparatus may be switched to the multi-lead measurement mode based on a switching control signal of the mode selection unit.

In this embodiment, the plurality of leads may be six leads. The signal measurement apparatus may include three measurement electrodes, and can obtain signals of six leads by using a Wilson network.

S102: A feature extraction module extracts features of the signals of the plurality of leads.

The obtained signals of the plurality of leads are complete electrocardio signals of the user. The electrocardio signals may be generally represented by an electrocardiogram. Each cardiac beat cycle in the electrocardiogram includes a series of regular waveforms, including a P wave, a QRS complex, and a T wave. A start point, end point, peak, trough, and interval of these waveforms record detailed information of a cardiac activity status, which provides an important analysis basis for cardiac disease diagnosis. Therefore, after the signals of the plurality of leads are obtained, the features of the signals of the plurality of leads may be extracted.

Specifically, waveform information such as a P wave, a PR interval, a QRS complex, a J point, an ST segment, a T wave, a U wave, and a QT interval in an ECG waveform of the signals of the plurality of leads is extracted, to obtain a feature, for example, an R-R interval (R-R interval, RRI) or a frequency domain, for disease diagnosis. The P wave represents excitement of an atrium, the first half represents excitement of a right atrium, and the second half represents excitement of a left atrium. The PR interval represents duration that is required when stimulation generated by a sinoatrial node reaches a ventricle through the atrium, an atrioventricular junction, and an atrioventricular bundle, and causes a ventricular muscle to start to excite. Therefore, the PR interval is also referred to as atrioventricular conduction duration. The QRS complex represents ventricular depolarization, with an excitement time limit less than 0.11 second. When conduction block of left and right bundle branches of a heart, or ventricular enlargement or hypertrophy, or the like occurs, the QRS complex is widened and deformed, and is prolonged in time limit. The J point is an intersection point between an end of a QRS wave and a start of the ST segment. The J point indicates that all ventricular myocytes are depolarized. It is duration during which all ventricular muscles are depolarized and repolarization does not yet start. At this time, ventricular muscles of all positions are in a depolarized state, and there is no potential difference between cells. Therefore, the ST segment should be normally on an equipotential line. When ischemia or necrosis appears in a cardiac muscle of a position, the ventricle still has a potential difference after completion of depolarization. At this time, the ST segment on the electrocardiogram shifts. A subsequent T wave represents repolarization of the ventricle. In an upward lead of a principal wave of the QRS wave, a direction of the T wave should be the same as that of the principal wave of the QRS wave. The U wave is related to the repolarization of the ventricle. The QT interval represents duration from the depolarization to the repolarization of the ventricle.

S 103: A signal quality obtaining module obtains quality of the signals of the plurality of leads based on the features of the signals of the plurality of leads.

The obtained quality of the signals of the plurality of leads determines whether the features of the signals of the plurality of leads can be used as a measurement result. Overall quality of the signals of the plurality of leads may be obtained based on a feature of a signal of each lead. For example, the quality of the signals of the plurality of leads may be obtained based on an average value of the features of the signals of all the leads.

S104: The mode selection switch determines whether the quality of the signals of the plurality of leads is greater than a first threshold. If the quality of the signals of the plurality of leads is greater than the first threshold, step S106 is performed. If the quality of the signals of the plurality of leads is less than or equal to a first threshold, step S105 is performed.

If the quality of the signals of the plurality of leads is greater than the first threshold, it is indicated that the quality of the signals of the plurality of leads is good, and the signals of the plurality of leads can be used as the measurement result. If the quality of the signals of the plurality of leads is less than or equal to the first threshold, it is indicated that the quality of the signals of the plurality of leads is poor, the signals of the plurality of leads cannot be used as the measurement result. The mode selection switch needs to perform mode switching. The first threshold may be set based on experience.

S105: The mode selection switch determines whether the user is in a static state. If the user is in the static state, step S106 is performed, If the user is in a moving state, step S107 is performed.

If the user is in the static state, the measured signals of the plurality of leads are stable, and the signals of the plurality of leads obtained through measurement in the multi-lead measurement mode can be used as the measurement result. However, if the user is in the moving state, common-mode interference is caused to measurement. In addition, when the quality of the signals of the plurality of leads is poor, measurement needs to be performed in a single-lead measurement mode with right leg drive.

It should be noted that there is no particular sequence between steps S105 and S104. To be specific, the mode selection switch may first determine whether the quality of the signals of the plurality of leads is greater than the first threshold and then determine whether the user is in the static state, or may first determine whether the user is in the static state, and then determine whether the quality of the signals of the plurality of leads is greater than the first threshold, or may simultaneously determine whether the quality of the signals of the plurality of leads is greater than the first threshold and whether the user is in the static state.

When the quality of the signals of the plurality of leads is greater than the first threshold or the user is in the static state, an operation in the multi-lead measurement mode continues to be performed. When the quality of the signals of the plurality of leads is less than or equal to the first threshold and the user is in the moving state, the mode selection switch switches to the single-lead mode with the right leg drive, to perform measurement.

S 106: An output module outputs the extracted features of the signals of the plurality of leads.

The extracted features of the signals of the plurality of leads may be output in a manner, for example, a voice or screen display.

S107: If the mode selection switch determines that the quality of the signals of the plurality of leads is less than or equal to the first threshold and the user is in the moving state, the mode selection switch switches to the single-lead mode with the right leg drive. The signal obtaining module obtains a signal of a single lead.

If the quality of the signals of the plurality of leads is less than or equal to the first threshold and the user is in the moving state, the mode selection switch switches to the single-lead mode with the right leg drive. The signal obtaining module obtains a signal of a single lead. Specifically, the signal obtaining module performs measurement by using other two electrodes other than the measurement electrode connected to the mode selection switch, to obtain the signal of the single lead.

S 108: A common-mode signal elimination module eliminates a common-mode signal from the signal of the single lead.

Common-mode components of electrical signals on surfaces of two electrodes are transmitted to the right leg drive circuit. A common-mode voltage of a human body is forced to approach a reference level by using a negative feedback of the right leg drive, so that centers of inputs of all the electrodes are located at a center of an input range, to reduce common-mode noise of a system. To be specific, the common-mode signal can be eliminated from the signal of the single lead, to improve signal quality of the system.

S109: The feature extraction module extracts a feature of the signal of the single lead on which elimination processing is performed.

Similar to that of extracting the features of the signals of the plurality of leads, the feature extraction module may extract the feature of the signal of the single lead on which elimination processing is performed.

Further, before the feature of the signal of the single lead is extracted, the signal of the single lead may be filtered.

S110: Output the extracted feature of the signal of the single lead.

The extracted feature of the signal of the single lead may be output in a manner, for example, a voice or screen display.

According to the signal measurement method provided in this embodiment of this application, automatic switching between a multi-lead ECG measurement mode and a single-lead ECG measurement mode with the right leg drive electrode of a three-electrode ECG system is implemented by determining the quality of the signals and a motion status of the user. When the quality of the signals of the plurality of leads is good, the extracted features of the signals of the plurality of leads are output. Alternatively, the feature of the signal of the single lead on which the elimination processing is performed is extracted and output only after the common-mode signal is eliminated from the signal of the single lead, so that an electrocardio signal with higher quality can be obtained, to accurately measure an electrocardio signal of the user.

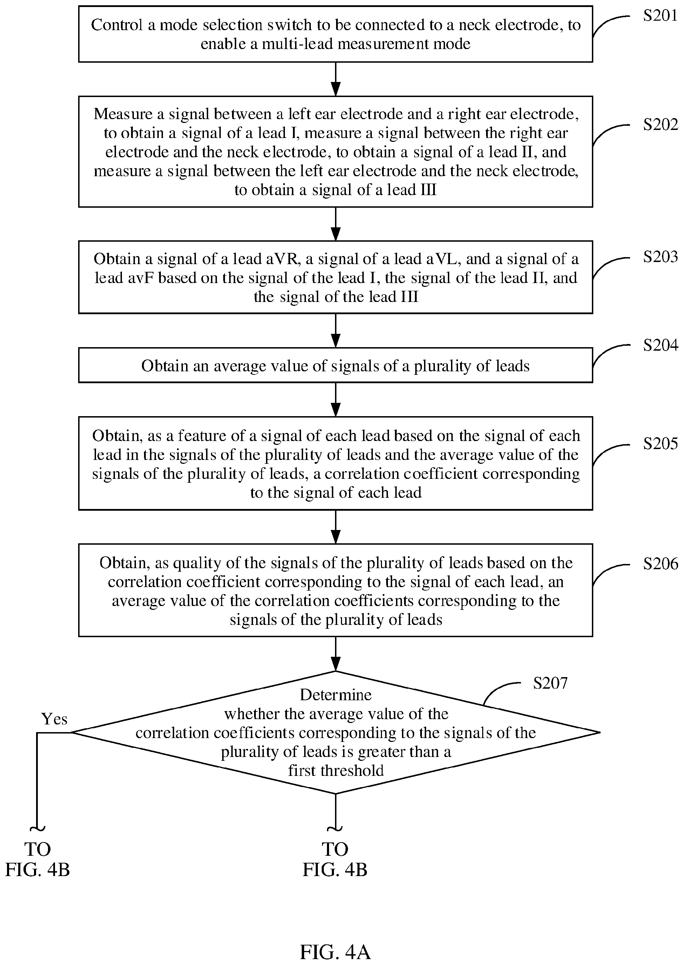

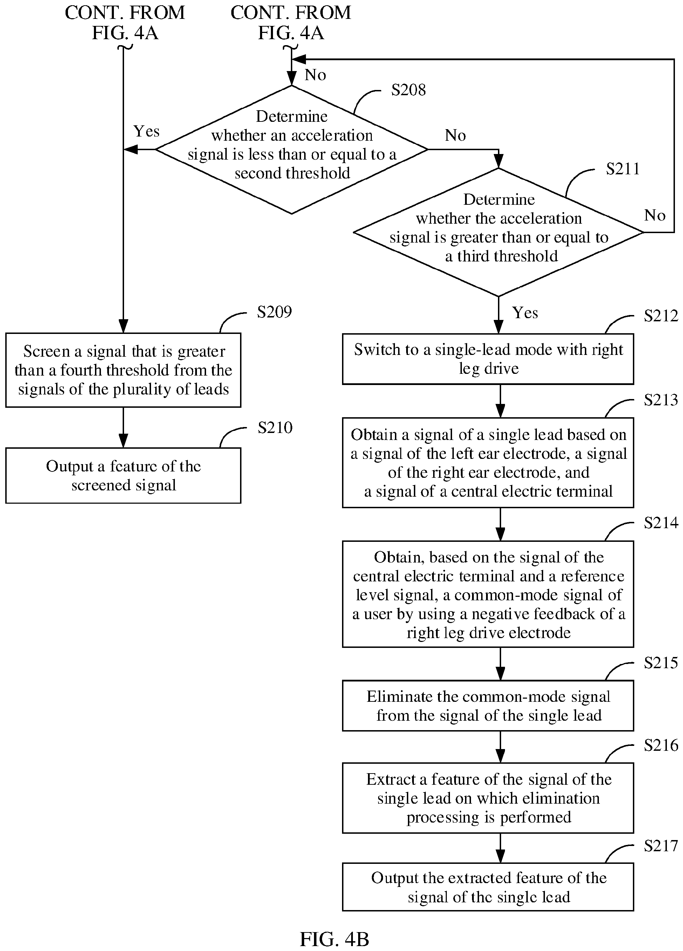

FIG. 4A and FIG. 4B are a schematic flowchart of another signal measurement method according to an embodiment of this application. The method may include the following steps.

S201: A mode selection switch is connected to a neck electrode, to enable a multi-lead measurement mode.

In this embodiment, a signal measurement apparatus may be the neckband headset shown in FIG. 1. The mode selection switch is connected to the neck electrode based on a user instruction or internal operation instructions, to enable the multi-lead measurement mode. When being connected to the neck electrode, the mode selection switch is disconnected from a right leg drive electrode.

S202: A signal obtaining module measures a signal between a left ear electrode and a right ear electrode, to obtain a signal of a lead I, measures a signal between the right ear electrode and the neck electrode, to obtain a signal of a lead II, and measures a signal between the left ear electrode and the neck electrode, to obtain a signal of a lead III.

S203: The signal obtaining module obtains a signal of a lead aVR, a signal of a lead aVL, and a signal of a lead avF based on the signal of the lead I, the signal of the lead II, and the signal of the lead III.

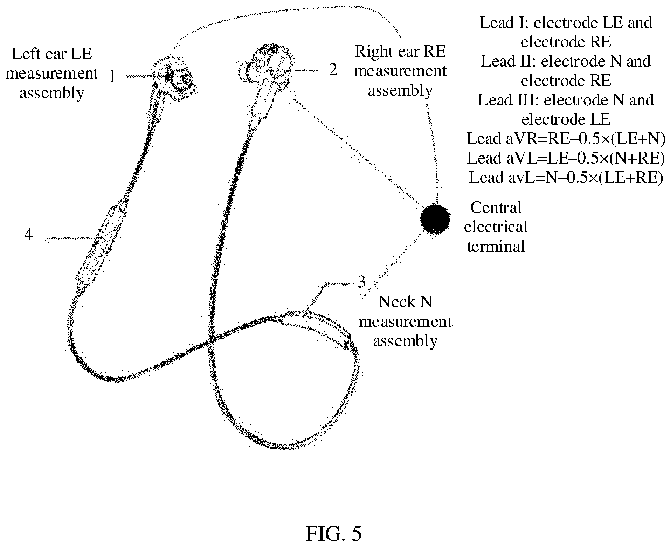

FIG. 5 is a schematic diagram of measurement in the multi-lead measurement mode by using the neckband headset shown in FIG. 1. The left ear LE electrode, the right ear RE electrode, and the neck N electrode are all connected to the central electrical terminal. The electrode LE and the electrode RE constitute the lead I, the electrode N and the electrode RE constitute the lead II, and the electrode N and the electrode LE constitute the lead III.

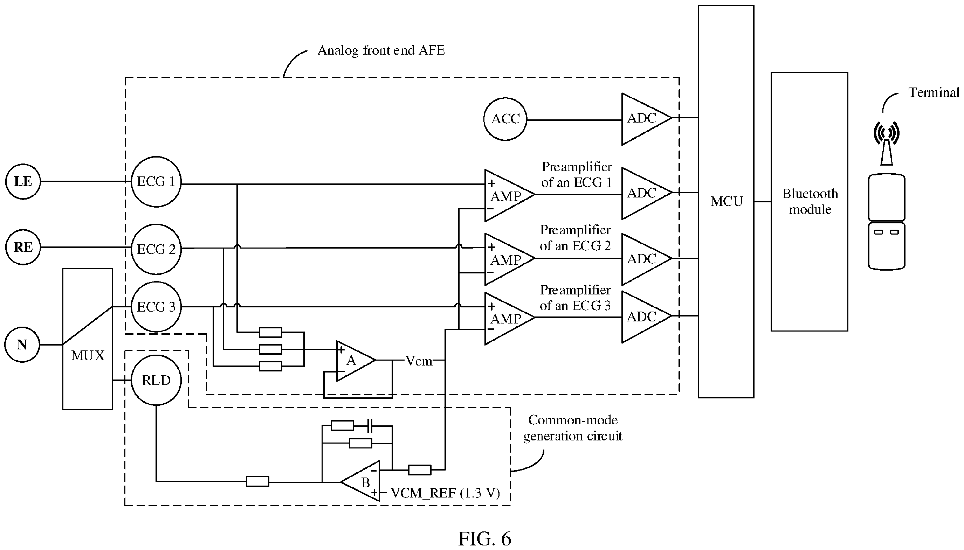

FIG. 6 is a schematic diagram of an example of an internal circuit of the signal measurement apparatus in the multi-lead measurement mode. Certainly, a circuit structure for signal measurement is not limited thereto. Electrocardio signals of a surface of skin are collected by using electrodes: the left ear (left ear, LE) electrode, the right ear (right ear, RE) electrode, and the neck (neck, N) electrode. Electrical signals of surfaces of the three electrodes pass through a multiplexer (multiplexer, MUX) circuit and a Wilson (Wilson) network to constitute a six-lead system, and are amplified and filtered by a two-stage gain-adjustable operational amplifier (for example, AD 761). Signals that are amplified and filtered, and are converted by an analog-to-digital-to-digital converter (analog-to-digital converter, ADC) are transmitted to an MCU. The Wilson network is a resistor network. The LE, RE, and N electrodes are connected together by using three equal resistors, to constitute a central terminal with an average potential, which is referred to as a Wilson central electrical terminal, a central potential terminal, or a Wilson central electric end. A voltage of the central electrical terminal represents an average voltage of a body. Then, an acceleration signal obtained through measurement by using an accelerometer (accelerometer, ACC) is processed by the ADC and transmitted to the MCU. The MCU processes the signals of the LE, RE, and N electrodes, and the central electrical terminal to obtain the signals of a plurality of leads. Specifically, during measurement mode selection and measurement, an internal switch of the multiplexer is switched to an ECG 3, so that the neck (N) electrode is connected to an input of the ECG 3 of an AFE module. In this case, the neck electrode is disconnected from a right leg drive circuit (RLD), and the right leg drive circuit does not work. The signals of the three electrodes are input to a positive electrode of an operational amplifier A. A human body common-mode voltage is input to the operational amplifier A through three electrode channels. The operational amplifier A performs an output to obtain Vcm of the Wilson central electrical terminal. Vcm is input to negative electrodes of preamplifiers of three ECGs (namely, a preamplifier of an ECG 1, a preamplifier of an ECG 2, and a preamplifier of the ECG 3). In this way, a six-lead system with three electrode inputs can be constituted.

Six leads refer to the lead I, the lead II, the lead III, the lead aVR, the lead aVL, and the lead avF. The electrode LE and the electrode RE constitute the lead I, the electrode N and the electrode RE constitute the lead II, and the electrode N and the electrode LE constitute the lead III. The lead aVR, the lead aVL, and the lead avF can be deduced based on the lead I, the lead II, and the lead III: lead aVR=RE-0.5×(LE+N), lead aVL=LE-0.5×(N+RE), and lead avF=N-0.5×(LE+RE).

Still refer to FIG. 5. The left ear LE electrode, the right ear RE electrode, and the neck N electrode are all connected to the central electrical terminal, the electrode LE and the electrode RE constitute the lead I, the electrode N and the electrode RE constitute the lead II, and the electrode N and the electrode LE constitute the lead III. The central electrical terminal is input to negative electrodes of the preamplifiers of three ECG electrodes, to constitute the six leads. To be specific, the lead aVR, the lead aVL, and the lead avF can be deduced based on the lead I, the lead II, and the lead III: lead aVR=RE-0.5×(LE+N), lead aVL=LE-0.5×(N+RE), and lead avF=N-0.5×(LE+RE).

S204: A feature extraction module obtains an average value of the signals of the plurality of leads.

S205: The feature extraction module obtains, as a feature of a signal of each lead based on the signal of each lead in the signals of the plurality of leads and the average value of the signals of the plurality of leads, a correlation coefficient corresponding to the signal of each lead.

The signals of the electrodes LE, RE, and N are input to the MCU, and the MCU may obtain signals A1 to A6 of the six leads based on a composition of the foregoing six leads. Further, after the signals of all the electrodes are obtained and before the signals are transmitted to the MCU, the signals may be filtered.

Feature extraction includes extracting correlation coefficients such as a Pearson correlation coefficient and a Spearman correlation coefficient from the signals of the plurality of leads. The correlation coefficient is a statistical indicator used to reflect closeness of correlation between variables. The Pearson correlation coefficient (Pearson correlation coefficient) is used to measure whether two data sets are on a same line, and is used to measure a linear relationship between fixed-range variables. In statistics, a Spearman's correlation coefficient for ranked data named after Charles Spearman is the Spearman correlation coefficient. The Spearman correlation coefficient is often expressed in the Greek letter ρ. The Spearman correlation coefficient is a nonparametric indicator that measures dependency between two variables. The Spearman correlation coefficient uses a monotonic equation to evaluate correlation between two statistical variables. If there is no duplicate value in data and when the two variables are completely monotonically correlated, the Spearman correlation coefficient is +1 or -1.

For an ECG signal, a higher correlation coefficient between a lead and an average value of the plurality of leads indicates better signal quality. Six correlation coefficients may be obtained through computing based on the signal of each lead and an average value of the signals of the six leads. An average value of the six leads is A=( | A1 | + | A2 | + | A3 | + | A4 | + | A5 | + | A6 | )/6. A correlation coefficient corresponding to A1 is γ₁=cov(A,A1)/σ_{A}σ_{A1}, and a correlation coefficient corresponding to A2 is γ₂=cov(A,A2)/σ_{A}σ_{A2}, and so on.

S206: A signal quality obtaining module obtains, as the quality of the signals of the plurality of leads based on the correlation coefficient corresponding to the signal of each lead, an average value of the correlation coefficients corresponding to the signals of the plurality of leads.

After the correlation coefficient of the signal of each lead is obtained, an average value Feat1=( | γ₁ | + | γ₂ | + | γ₃ | + | γ₄ | + | γ₅ | + | γ₆ | )/6 of the six correlation coefficients may be obtained, and reflects overall signal quality of the signals of the plurality of leads.

It may be understood that the quality of the signals may be alternatively determined based on the signals of the plurality of leads. The quality of the signals may be a specific signal value.

S207: The mode selection switch determines whether the average value of the correlation coefficients corresponding to the signals of the plurality of leads is greater than a first threshold. If the average value is greater than the first threshold, step S209 is performed. If the average value is not greater than the first threshold, step S208 is performed.

The mode selection switch determines whether the quality of the signals of the plurality of leads is greater than the first threshold. Specifically, the mode selection switch determines, based on the obtained average value of the correlation coefficients corresponding to the signals of the plurality of leads, whether the average value is greater than the first threshold.

S208: The determining module determines whether an acceleration signal is less than or equal to a second threshold. If the acceleration signal is less than or equal to the second threshold, step S209 is performed. If the acceleration signal is greater than the second threshold, step S211 is performed.

Whether the user is in a static state or a moving state may be determined by using an acceleration signal of the apparatus. In this case, the signal obtaining module may further obtain the acceleration signal. Specifically, an acceleration signal of the user may be collected by using an acceleration sensor. The acceleration signal is transmitted to the MCU. The acceleration sensor may be specifically a gravity sensor or the like. When the user wears the headset and is in the moving state, the neckband headset uses a characteristic that crystal deformation inside the gravity sensor of the neckband headset is caused by an acceleration. Because the deformation generates a voltage, as long as a relationship between the generated voltage and the applied acceleration is computed, the acceleration may be converted into the voltage for output.

When the acceleration signal is less than or equal to the second threshold, the determining module determines that the user is in the static state. When the acceleration signal is greater than or equal to a third threshold, the determining module determines that the user is in the moving state. The determining module outputs a determined result to the mode selection switch.

It should be noted that there is no sequence between S207 and S208. When the average value of the correlation coefficients corresponding to the signals of the plurality of leads or the acceleration signal is less than or equal to the second threshold, S209 is performed.

S209: If the average value of the correlation coefficients corresponding to the signals of the plurality of leads is greater than the first threshold, or the acceleration signal is less than or equal to the second threshold, a signal screening module screens a signal that is greater than a fourth threshold from the signals of the plurality of leads.

If the quality of the signals of the plurality of leads is greater than the first threshold, it is indicated that the obtained overall signal quality of a multi-lead system is greater than the first threshold, and the overall signal quality is good. Alternatively, if the acceleration signal is less than or equal to the second threshold, it is indicated that the user is basically in the static state currently, and the quality of the signals of the plurality of leads obtained by using the multi-lead system is good.

When it is determined that the signals of the plurality of leads can be output, a desired signal of a lead may be screened based on a requirement. For example, for a disease that can be diagnosed only by observing a specific lead, for example, a coronary heart disease, the desired signal of the lead is screened. For another example, the signal of each lead is compared with the fourth threshold, and a lead signal that is greater than or equal to the fourth threshold is screened.

Further, the screened lead signal may be filtered.

S210: Output a feature of the screened signal.

Because the feature of the signal of each lead in the signals of the plurality of leads has been extracted previously, the feature of the screened signal can be output based on the screened lead signal.

S211: The determining module determines whether the acceleration signal is greater than or equal to the third threshold. If the acceleration signal is greater than or equal to the third threshold, step S212 is performed. If the acceleration signal is less than the third threshold, step S208 is performed.

If the average value of the correlation coefficients corresponding to the signals of the plurality of leads is less than or equal to the first threshold, that is, the quality of the signals of the plurality of leads is poor, and the determining module determines that the acceleration signal is greater than or equal to the third threshold, that is, the user is in the moving state, the determined result is output to the mode switching switch.

S212: The mode switching switch switches to a single-lead mode with right leg drive.

The mode switching switch switches to the single-lead mode with the right leg drive when it is determined that the quality of the signals of the plurality of leads is poor and the user is in the moving state.

S213: The signal obtaining module obtains a signal of a single lead based on a signal of the left ear electrode, a signal of the right ear electrode, and a signal of the central electric terminal.

S214: Obtain, based on the signal of the central electric terminal and a reference level signal, a common-mode signal of the user by using a negative feedback of the right leg drive electrode.

S215: Eliminate the common-mode signal from the signal of the single lead.

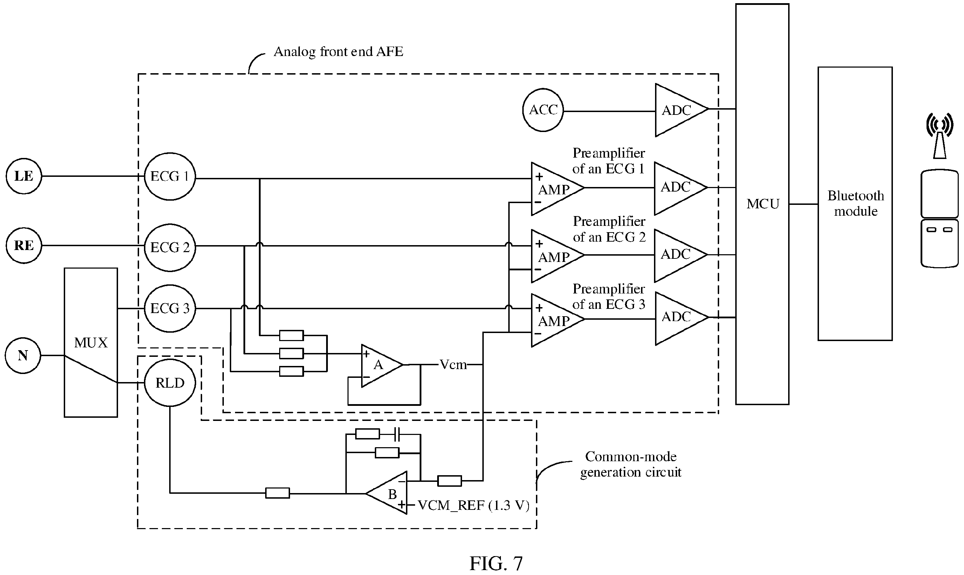

Specifically, FIG. 7 is a schematic diagram of an example of a structure of an internal circuit of the signal measurement apparatus in a single-lead measurement mode. If the quality of the signals of the plurality of leads is less than or equal to the first threshold and the acceleration signal is greater than or equal to the second threshold, the internal switch of the multiplexer is switched to the RLD, so that the neck (N) electrode is connected to the RLD of the AFE module, and is disconnected from the ECG 3 of the AFE module at the same time, and an ECG 3 channel of the AFE module does not work. The signals of the two electrodes (LE and RE) are input to a positive electrode of a preamplifier of an ECG. A common-mode voltage (common-mode components of electrical signals on surfaces of the two electrodes) of the human body is input to the operational amplifier A through two electrode channels (LE and RE). The operational amplifier A performs an output to obtain Vcm of the Wilson central electrical terminal. Vcm is input to negative electrodes of preamplifiers of two ECGs, in addition, is input to a negative electrode of an operational amplifier B of the right leg drive circuit, and is fed back to the human body through the right leg drive circuit. A reference level of 1.3 V is input to a positive electrode of the operational amplifier B, the common-mode voltage of the human body is forced to approach the reference level by using a negative feedback of the right leg drive, so that centers of inputs of all the electrodes are located at a center of an input range, to constitute a single-lead circuit with the "right leg drive". In this way, the common-mode signal can be eliminated from the signal of the single lead, to obtain the signal of the single lead with good quality.

S216: Extract a feature of the signal of the single lead on which elimination processing is performed.

After the common-mode signal is eliminated from the signal of the single lead, the feature of the signal of the single lead on which the elimination processing is performed may be extracted. For an extraction process, refer to steps S204 and S205.

S217: Output the extracted feature of the signal of the single lead.

According to the signal measurement method provided in this embodiment of this application, automatic switching between a multi-lead ECG measurement mode and a single-lead ECG measurement mode with the right leg drive electrode of a three-electrode ECG system is implemented by determining the quality of the signals and a motion status of the user. When the quality of the signals of the plurality of leads is good, the extracted features of the signals of the plurality of leads are output. Alternatively, the feature of the signal of the single lead on which the elimination processing is performed is extracted and output only after the common-mode signal is eliminated from the signal of the single lead, so that an electrocardio signal with higher quality can be obtained, to accurately measure an electrocardio signal of the user.

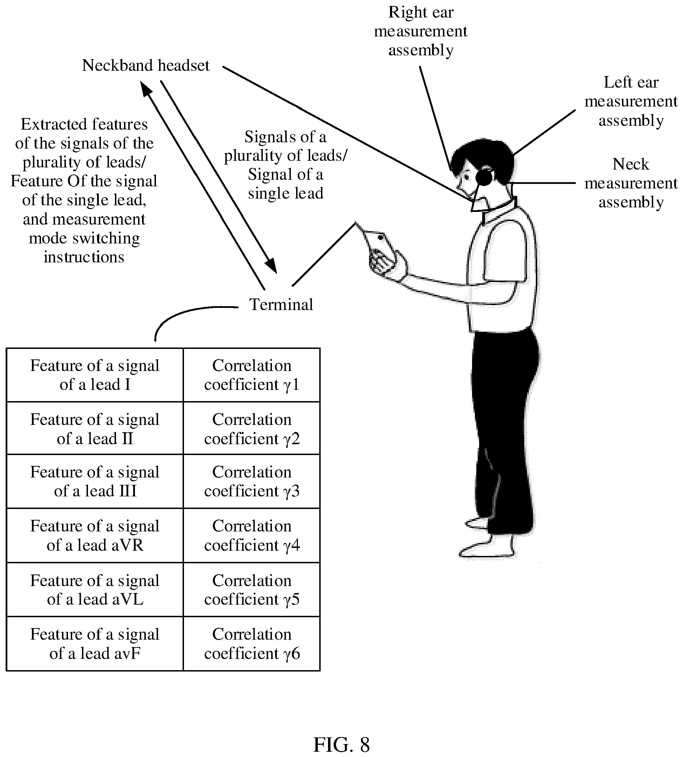

FIG. 8 is a schematic diagram of a scenario in which a user performs measurement by using a signal measurement apparatus. With reference to the foregoing descriptions, in an actual use process, when the user wears the foregoing neckband headset and turns on the headset to measure an electrocardio signal, the headset enters a multi-lead measurement mode by default. In addition, an acceleration sensor in the headset is used to measure an acceleration signal. When it is determined that the user is in a relatively static state, signals obtained through measurement by using a left ear electrode, a right ear electrode, and a neck electrode are processed, to obtain signals of a plurality of leads. When quality of the signals of the plurality of leads is good or the user is in the relatively static state, features of the signals of the plurality of leads are extracted. If the quality of the signals of the plurality of leads is poor or the user is in a moving state, the headset may be switched to a single-lead measurement mode. In this case, the neck electrode is used as a right leg drive electrode, and common-mode components of electrical signals on surfaces of the left ear electrode and the right ear electrode are transmitted to the right leg drive electrode. A common-mode voltage of a human body is forced to approach a reference level by using a negative feedback of right leg drive, so that centers of inputs of all the electrodes are located at a center of an input range, to reduce common-mode noise of a system and improve signal quality of a signal of a single lead.

After the headset obtains the signals of the electrodes through measurement, the signals may be processed inside the headset to obtain the signals of the plurality of leads or the signal of the single lead, and a feature of a lead signal is extracted. In addition, the feature of the lead signal may be output by using a headset player. After obtaining the signals of the electrodes through measurement, the headset may alternatively transmit the signals to a terminal that establishes a wireless connection to the headset, for example, a mobile phone or another wearable device of the user. The mobile phone or the another wearable device processes the signals to obtain the signals of the plurality of leads or the signal of the single lead, extracts the feature of the lead signal, and outputs the feature by the mobile phone or the another wearable device. In this way, requirements on a computing capability, a signal output capability, and the like of the signal measurement apparatus can be reduced.

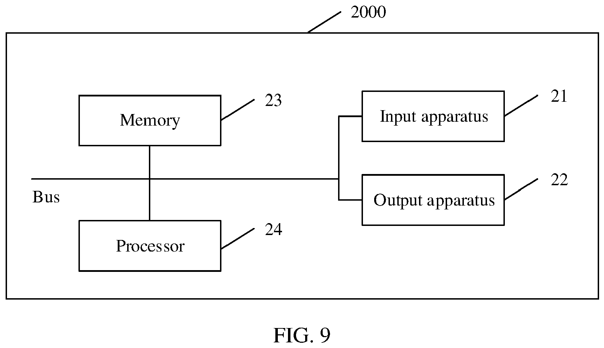

FIG. 9 is a schematic diagram of a structure of a signal measurement apparatus. The signal measurement apparatus is configured to perform the foregoing signal measurement methods. Some or all of the foregoing methods may be implemented by using hardware, or may be implemented by using software or firmware.

Optionally, the signal measurement apparatus may be a chip or an integrated circuit in a specific implementation.

Optionally, when some or all of the signal measurement methods in the foregoing embodiments are implemented by using software or firmware, a signal measurement apparatus 2000 provided in FIG. 9 may be used for implementation. As shown in FIG. 9, the signal measurement apparatus 2000 may include:
a memory 23 and a processor 24 (where there may be one or more processors 24 in the apparatus, and an example in which there is one processor is used in FIG. 9), and may further include an input apparatus 21 and an output apparatus 22. In this embodiment, the input apparatus 21, the output apparatus 22, the memory 23, and the processor 24 may be connected through a bus or in another manner. A connection through the bus is used as an example in FIG. 9.

The processor 24 is configured to perform the method steps performed in FIG. 3 and FIG. 4A and FIG. 4B.

Specifically, the processor 24 is configured to invoke program instructions to perform the following operations:
enabling a multi-lead measurement mode, and obtaining signals of a plurality of leads and a status of a user; extracting features of the signals of the plurality of leads, and obtaining quality of the signals of the plurality of leads based on the features of the signals of the plurality of leads; and controlling the output apparatus to output the extracted features of the signals of the plurality of leads if the quality of the signals of the plurality of leads is greater than a first threshold or the user is in a static state; or switching to a single-lead mode with right leg drive if the quality of the signals of the plurality of leads is less than or equal to a first threshold and the user is in a moving state, and obtaining a signal of a single lead; eliminating a common-mode signal from the signal of the single lead; and extracting a feature of the signal of the single lead on which elimination processing is performed, and controlling the output apparatus to output the extracted feature of the signal of the single lead.

In a possible implementation, the processor 24 is further configured to perform the following operation: controlling a mode selection switch to be connected to a neck electrode, to enable the multi-lead measurement mode.

In another possible implementation, that the processor 24 performs the operation of obtaining signals of a plurality of leads includes: measuring a signal between a left ear electrode and a right ear electrode, to obtain a signal of a lead I; measuring a signal between the right ear electrode and the neck electrode, to obtain a signal of a lead II; measuring a signal between the left ear electrode and the neck electrode, to obtain a signal of a lead III; and obtaining a signal of a lead aVR, a signal of a lead aVL, and a signal of a lead avF based on the signal of the lead I, the signal of the lead II, and the signal of the lead III.

In still another possible implementation, that the processor 24 performs the operation of obtaining a signal of a lead aVR, a signal of a lead aVL, and a signal of a lead avF based on the signal of the lead I, the signal of the lead II, and the signal of the lead III includes: The signal of the lead aVR satisfies: lead aVR=RE-0.5×(LE+N), the signal of the lead aVL satisfies: lead aVL=LE-0.5×(N+RE), and the signal of the lead avF satisfies: lead avF=N-0.5×(LE+RE). RE is a signal of the right ear electrode, LE is a signal of the left ear electrode, and N is a signal of the neck electrode.

In still another possible implementation, that the processor 24 performs the operation of extracting features of the signals of the plurality of leads includes: obtaining an average value of the signals of the plurality of leads; and obtaining, as a feature of a signal of each lead based on the signal of each lead in the signals of the plurality of leads and the average value of the signals of the plurality of leads, a correlation coefficient corresponding to the signal of each lead.

In still another possible implementation, the processor 24 further performs the following operations: obtaining, as the quality of the signals of the plurality of leads based on the correlation coefficient corresponding to the signal of each lead, an average value of the correlation coefficients corresponding to the signals of the plurality of leads; and performing the operation of extracting the features of the signals of the plurality of leads if the average value of the correlation coefficients corresponding to the signals of the plurality of leads is greater than the first threshold or an acceleration signal is less than or equal to a second threshold; or performing the operation of switching to the single-lead mode with the right leg drive if the average value of the correlation coefficients corresponding to the signals of the plurality of leads is less than or equal to the first threshold and an acceleration signal is greater than or equal to a third threshold.

In still another possible implementation, the processor 24 further performs the following operations: screening a signal that is greater than a fourth threshold from the signals of the plurality of leads; and extracting a feature of the screened signal.

In still another possible implementation, that the processor 24 performs the operation of: switching to a single-lead mode with the right leg drive, and obtaining a signal of a single lead if the signals of the plurality of leads are less than or equal to a first threshold and the user is in a moving state includes: if the signals of the plurality of leads are less than or equal to the first threshold and the user is in the moving state, controlling the mode selection switch to switch to a right leg drive electrode, to enable the single-lead mode. That the processor 24 performs the operation of obtaining a signal of a single lead includes: obtaining the signal of the single lead based on the signal of the left ear electrode, the signal of the right ear electrode, and a signal of a central electric terminal. That the processor 24 performs the operation of eliminating a common-mode signal from the signal of the single lead includes: obtaining, based on the signal of the central electric terminal and a reference level signal, a common-mode signal of the user by using a negative feedback of the right leg drive electrode; and eliminating the common-mode signal from the signal of the single lead.

In still another possible implementation, that the processor 24 performs the operation of obtaining a status of a user includes: obtaining the acceleration signal; and if the acceleration signal is less than or equal to the second threshold, determining that the user is in the static state; or if the acceleration signal is greater than or equal to the third threshold, determining that the user is in the moving state.

Optionally, a program for the signal measurement method may be stored in the memory 23. The memory 23 may be a physically independent unit, or may be integrated with the processor 24. The memory 23 may be also configured to store data.

Optionally, when some or all of the signal measurement methods in the foregoing embodiments are implemented by using software, the signal measurement apparatus may alternatively include only a processor. A memory configured to store a program is located outside the signal measurement apparatus. The processor is connected to the memory by using a circuit or a wire, and is configured to read and execute the program stored in the memory.

The processor may be a central processing unit (central processing unit, CPU), a network processor (network processor, NP), or a WLAN device.

The processor may further include a hardware chip. The hardware chip may be an application-specific integrated circuit (application-specific integrated circuit, ASIC), a programmable logic device (programmable logic device, PLD), or a combination thereof. The PLD may be a complex programmable logic device (complex programmable logic device, CPLD), a field-programmable gate array (field-programmable gate array, FPGA), generic array logic (generic array logic, GAL), or any combination thereof.

The memory may include a volatile memory (volatile memory), for example, a random access memory (random access memory, RAM). The memory may include a non-volatile memory (non-volatile memory), for example, a flash memory (flash memory), a hard disk drive (hard disk drive, HDD), or a solid-state drive (solid-state drive, SSD). The memory may alternatively include a combination of the foregoing types of memories.

It may be clearly understood by a person skilled in the art that, for the purpose of convenient and brief description, for a detailed working process of the foregoing system, apparatus, and unit, refer to a corresponding process in the foregoing method embodiments. Details are not described herein again.

In the several embodiments provided in this application, it should be understood that the disclosed system, apparatus, and method may be implemented in other manners. For example, division into the units is merely a logical function division and may be another division during actual implementation. For example, a plurality of units or assemblies may be combined or integrated into another system, or some features may be ignored or may not be performed. The displayed or discussed mutual couplings or direct couplings or communication connections may be implemented through some interfaces. The indirect couplings or communication connections between the apparatuses or units may be implemented in an electrical form, a mechanical form, or another form.

The units described as separate parts may or may not be physically separate, and parts displayed as units may or may not be physical units, that is, may be located in one position, or may be distributed on a plurality of network units. Some or all of the units may be selected based on actual requirements to achieve the objectives of the solutions in embodiments.

All or some of the foregoing embodiments may be implemented by using software, hardware, firmware, or any combination thereof. When software is used to implement the embodiments, all or some of the embodiments may be implemented in a form of a computer program product. The computer program product includes one or more computer instructions. When the computer program instructions are loaded and executed on a computer, all or some of the procedures or functions in embodiments of this application are generated. The computer may be a general-purpose computer, a dedicated computer, a computer network, or another programmable apparatus. The computer instructions may be stored in a computer-readable storage medium, or may be transmitted by using a computer-readable storage medium. The computer instructions may be transmitted from a website, computer, server, or data center to another website, computer, server, or data center in a wired (for example, a coaxial cable, an optical fiber, or a digital subscriber line (digital subscriber line, DSL)) or wireless (for example, infrared, radio, or microwave) manner. The computer-readable storage medium may be any usable medium accessible by a computer, or a data storage device, for example, a server or a data center, integrating one or more usable media. The usable medium may be a read-only memory (read-only memory, ROM), or a random access memory (random access memory, RAM), or a magnetic medium, for example, a floppy disk, a hard disk drive, a magnetic tape, or a magnetic disk, or an optical medium, for example, a digital versatile disc (digital versatile disc, DVD), or a semiconductor medium, for example, a solid-state drive (solid-state disk, SSD).

## Claims

1. A signal measurement method, applied to a signal measurement apparatus, wherein the method comprises:
enabling a multi-lead measurement mode, and obtaining signals of a plurality of leads and a status of a user;
extracting features of the signals of the plurality of leads, and obtaining quality of the signals of the plurality of leads based on the features of the signals of the plurality of leads; and
outputting the extracted features of the signals of the plurality of leads if the quality of the signals of the plurality of leads is greater than a first threshold or the user is in a static state; or
switching to a single-lead mode with right leg drive if the quality of the signals of the plurality of leads is less than or equal to a first threshold and the user is in a motion state, and obtaining a signal of a single lead;
eliminating a common-mode signal from the signal of the single lead; and
extracting and outputting a feature of the signal of the single lead on which elimination processing is performed.

2. The method according to claim 1, wherein the signal measurement apparatus comprises a left ear electrode, a right ear electrode, a neck electrode, preamplifiers respectively corresponding to the three electrodes, a Wilson network circuit, a common-mode generation circuit, a mode selection switch, and a processor, the left ear electrode is connected to a left ear measurement assembly, the right ear electrode is connected to a right ear measurement assembly, the mode selection switch is connected to a neck measurement assembly, and the common-mode generation circuit comprises a right leg drive electrode and a common-mode amplifier;
the left ear electrode, the right ear electrode, and the neck electrode are respectively and separately connected to positive electrodes of the corresponding preamplifiers and the Wilson network circuit, a central electrical terminal of the Wilson network circuit is separately connected to negative electrodes of the preamplifiers of the three electrodes, and the preamplifiers respectively corresponding to the three electrodes are connected to the processor;
the central electrical terminal of the Wilson network circuit is further connected to a negative electrode of the common-mode amplifier; and
the enabling a multi-lead measurement mode comprises:
controlling the mode selection switch to be connected to the neck electrode, to enable the multi-lead measurement mode.

3. The method according to claim 2, wherein the obtaining signals of a plurality of leads comprises:
measuring a signal between the left ear electrode and the right ear electrode, to obtain a signal of a lead I;
measuring a signal between the right ear electrode and the neck electrode, to obtain a signal of a lead II;
measuring a signal between the left ear electrode and the neck electrode, to obtain a signal of a lead III; and
obtaining a signal of a lead aVR, a signal of a lead aVL, and a signal of a lead avF based on the signal of the lead I, the signal of the lead II, and the signal of the lead III.

4. The method according to claim 3, wherein the obtaining a signal of a lead aVR, a signal of a lead aVL, and a signal of a lead avF based on the signal of the lead I, the signal of the lead II, and the signal of the lead III comprises:
the signal of the lead aVR satisfies: lead aVR=RE-0.5×(LE+N);
the signal of the lead aVL satisfies: lead aVL=LE-0.5×(N+RE); and
the signal of the lead avF satisfies: lead avF=N-0.5×(LE+RE), wherein
RE is a signal of the right ear electrode, LE is a signal of the left ear electrode, and N is a signal of the neck electrode.

5. The method according to claim 3 or 4, wherein the extracting features of the signals of the plurality of leads comprises:
obtaining an average value of the signals of the plurality of leads; and
obtaining, as a feature of a signal of each lead based on the signal of each lead in the signals of the plurality of leads and the average value of the signals of the plurality of leads, a correlation coefficient corresponding to the signal of each lead.

6. The method according to any one of claims 1 to 5, wherein the obtaining quality of the signals of the plurality of leads based on the features of the signals of the plurality of leads comprises:
obtaining, as the quality of the signals of the plurality of leads based on the correlation coefficient corresponding to the signal of each lead, an average value of the correlation coefficients corresponding to the signals of the plurality of leads; and
performing the step of outputting the features of the signals of the plurality of leads if the average value of the correlation coefficients corresponding to the signals of the plurality of leads is greater than the first threshold or an acceleration signal is less than or equal to a second threshold; or
performing the step of switching to the single-lead mode with the right leg drive if the average value of the correlation coefficients corresponding to the signals of the plurality of leads is less than or equal to the first threshold and an acceleration signal is greater than or equal to a third threshold.

7. The method according to any one of claims 1 to 6, wherein the method further comprises:
screening a signal that is greater than a fourth threshold from the signals of the plurality of leads; and
extracting a feature of the screened signal.

8. The method according to any one of claims 2 to 7, wherein the switching to a single-lead mode with right leg drive if the quality of the signals of the plurality of leads is less than or equal to a first threshold and the user is in a motion state, and obtaining a signal of a single lead comprises:
if the quality of the signals of the plurality of leads is less than or equal to the first threshold and the user is in the moving state, controlling the mode selection switch to switch to the right leg drive electrode, to enable the single-lead mode;
the obtaining a signal of a single lead comprises:
obtaining the signal of the single lead based on the signal of the left ear electrode, the signal of the right ear electrode, and a signal of the central electric terminal; and
the eliminating a common-mode signal from the signal of the single lead comprises:
obtaining, based on the signal of the central electric terminal and a reference level signal, a common-mode signal of the user by using a negative feedback of the right leg drive electrode; and
eliminating the common-mode signal from the signal of the single lead.

9. The method according to claim 6, wherein the obtaining a status of a user comprises:
obtaining the acceleration signal; and
if the acceleration signal is less than or equal to the second threshold, determining that the user is in the static state; or
if the acceleration signal is greater than or equal to the third threshold, determining that the user is in the moving state.

10. A signal measurement apparatus, wherein the apparatus comprises:
a mode selection switch, configured to enable a multi-lead measurement mode;
a signal obtaining module, configured to obtain signals of a plurality of leads and a status of a user;
a feature extraction module, configured to extract features of the signals of the plurality of leads;
a signal quality obtaining module, configured to obtain quality of the signals of the plurality of leads based on the features of the signals of the plurality of leads;
an output module, configured to output the extracted features of the signals of the plurality of leads if the quality of the signals of the plurality of leads is greater than a first threshold or the user is in a static state, wherein
the mode selection switch is further configured to switch to a single-lead mode with right leg drive if the quality of the signals of the plurality of leads is less than or equal to a first threshold and the user is in a moving state; and
the signal obtaining module is further configured to obtain a signal of a single lead; and
a common-mode signal elimination module, configured to eliminate a common-mode signal from the signal of the single lead, wherein
the feature extraction module is further configured to extract a feature of the signal of the single lead on which elimination processing is performed; and
the output module is further configured to output the extracted feature of the signal of the single lead.

11. The apparatus according to claim 10, wherein the signal obtaining module comprises a left ear electrode, a right ear electrode, a neck electrode, preamplifiers respectively corresponding to the three electrodes, a Wilson network circuit, and a processor, the common-mode signal elimination module comprises a right leg drive electrode and a common-mode amplifier, the left ear electrode is connected to a left ear measurement assembly, the right ear electrode is connected to a right ear measurement assembly, the mode selection switch is connected to a neck measurement assembly, the left ear electrode, the right ear electrode, and the neck electrode are respectively and separately connected to positive electrodes of the corresponding preamplifiers and the Wilson network circuit, a central electrical terminal of the Wilson network circuit is separately connected to negative electrodes of the preamplifiers of the three electrodes, the preamplifiers respectively corresponding to the three electrodes are connected to the processor, and the central electrical terminal of the Wilson network circuit is further connected to a negative electrode of the common-mode amplifier; and
the module selection switch is configured to connect to the neck electrode, to enable the multi-lead measurement mode.

12. The apparatus according to claim 10 or 11, wherein the signal obtaining module is configured to:
measure a signal between the left ear electrode and the right ear electrode, to obtain a signal of a lead I;
measure a signal between the right ear electrode and the neck electrode, to obtain a signal of a lead II;
measure a signal between the left ear electrode and the neck electrode, to obtain a signal of a lead III; and
obtain a signal of a lead aVR, a signal of a lead aVL, and a signal of a lead avF based on the signal of the lead I, the signal of the lead II, and the signal of the lead III.

13. The apparatus according to claim 12, wherein the signal of the lead aVR satisfies: lead aVR=RE-0.5×(LE+N);
the signal of the lead aVL satisfies: lead aVL=LE-0.5×(N+RE); and
the signal of the lead avF satisfies: lead avF=N-0.5×(LE+RE), wherein
RE is a signal of the right ear electrode, LE is a signal of the left ear electrode, and N is a signal of the neck electrode.

14. The apparatus according to any one of claims 10 to 13, wherein the feature extraction module is configured to obtain an average value of the signals of the plurality of leads; and obtain, as a feature of a signal of each lead based on the signal of each lead in the signals of the plurality of leads and the average value of the signals of the plurality of leads, a correlation coefficient corresponding to the signal of each lead.

15. The apparatus according to any one of claims 10 to 14, wherein the signal quality obtaining module is configured to obtain, as the quality of the signals of the plurality of leads based on the correlation coefficient corresponding to the signal of each lead, an average value of the correlation coefficients corresponding to the signals of the plurality of leads;
the output module is configured to output the features of the signals of the plurality of leads if the average value of the correlation coefficients corresponding to the signals of the plurality of leads is greater than the first threshold or an acceleration signal is less than or equal to a second threshold; and
the mode selection switch is configured to switch to the single-lead mode with the right leg drive if the average value of the correlation coefficients corresponding to the signals of the plurality of leads is less than or equal to the first threshold and the acceleration signal is greater than or equal to a third threshold.

16. The apparatus according to any one of claims 10 to 15, wherein the apparatus further comprises:
a signal screening module, configured to screen a signal that is greater than a fourth threshold from the signals of the plurality of leads; and
the feature extraction module is configured to extract a feature of the screened signal.

17. The apparatus according to any one of claims 11 to 16, wherein the mode selection switch is configured to switch to the right leg drive electrode if the signals of the plurality of leads are less than or equal to the first threshold and the user is in the moving state, to enable the single-lead mode;
the signal obtaining module is configured to obtain the signal of the single lead based on the signal of the left ear electrode, the signal of the right ear electrode, and a signal of the central electric terminal; and
the common-mode signal elimination module is configured to obtain, based on the signal of the central electric terminal and a reference level signal, a common-mode signal of the user by using a negative feedback of the right leg drive electrode, and eliminate the common-mode signal from the signal of the single lead.

18. The apparatus according to claim 15, wherein the signal obtaining module is further configured to obtain the acceleration signal; and
the apparatus further comprises:
a determining module, configured to: if the acceleration signal is less than or equal to the second threshold, determine that the user is in the static state, wherein
the determining module is further configured to: if the acceleration signal is greater than or equal to the third threshold, determine that the user is in the moving state.
